# EUROPEAN PATENT APPLICATION

(11) **EP 1 400 809 A1**
(43) Date of publication of application: **24.03.2004**
(21) Application number: 03020492.9
(22) Date of filing: 15.09.2003
(51) Int. Cl.: G01N 33/68, C07K 14/315

(54) **Test for caries susceptibility: detection of the IgA levels in saliva specimens**

(30) Priority: 19.09.2002 JP 2002273125
(71) Applicant: GC Corporation, Itabashi-ku, Tokyo 174 (JP)
(72) Inventor: Senpuku, Hidenobu, Yokohama-shi Kanagawa-ken (JP); Masuzawa, Yumiko, Tokyo (JP); Okada, Junichi, Tokyo (JP)
(74) Representative: Müller-Boré & Partner Patentanwälte

(57) **Abstract**

To accurately examine a caries risk in a short time, a synthetic peptide having the amino acid sequence composed of the formula of Asn Ala Lys Ala Thr Tyr Glu Ala Ala Leu Lys Gln Tyr Glu Ala Asp Leu Ala Ala Val Lys Lys Ala Asn Ala Ala is used as an antigen i.e. the fragment 361-386 of the surface protein antigen from S. mutans, and an antibody value of secretory immunoglobulin A in human saliva against said antigen is measured.

## Description

The present invention relates to a method for examining a caries risk, using a synthetic peptide having a specific amino acid sequence as an antigen, and measuring an antibody value of secretory immunoglobulin A (sIgA) contained in human saliva against said antigen by an immunological method.

It has been known that there are close relation between existence of mutans Streptococcus in a human oral cavity and generation of the caries, and many researches have been reported (for example, refer to a non-patent reference, Anders Thylstrup and Ole Fejerskov, "Textbook of clinical caliology" (Denmark), Munksgaard Company, 1996, 2_{nd} edition, p.405). Said mutans streptococci existing in the human saliva is the general term of Streptococcus mutans and Streptococcus sobrinus (hereinafter, they are referred to as "S. mutans" and "S. sobrinus" respectively) as strains.

Since it has been reported that the new caries may be generated further in the future if there is much quantity of the mutans streptococci existing in the human saliva (for example, refer to a non-patent reference, Zickert I, Emilson CG, Krasse B. "Effect of caries preventive measures in children highly infected with the bacterium Streptocuccus mutans" Arch Oral Biol, (USA), Oxford Pergamon Press, 1982, 27, p.861-8), many trials for easily determining the quantity of the mutans streptococci in the human saliva have been carried out. For example, there are the trials that the mutans streptococci quantity is determined by applying monoclonal antibody specifically reacted with the S. mutans, (for example, refer to Japanese Patent Laid-Open No. 177898-1990 and Japanese Patent Laid-Open No. 36400-1998), and that a bacterial cell itself grown in a simplified culturing kit is determined by visual observation, (for example, refer to a non-patent reference, McGhee J. R. et al, Adv. Esp. Med. Biol. (USA), Plenum Press, 1978, 107, p.177-184) . However, as for examining the caries risk by measuring the quantity of the mutans streptococci in the human saliva, there are the following problems. First, the quantity of the mutans streptococci in the human saliva is not always fixed. For example, if a subject brushes his teeth finely just before examining the quantity of the mutans streptococci in the human saliva, the quantity of the mutans streptococci is decreased temporarily, so that it is judged that he has low caries risk.

As it has been known that an increasing ratio of the quantity of the mutans streptococci is varied with persons, if the difference of said increasing processes can be examined beforehand, the caries risk can be known irrespective of the actual increasing or quantity of the mutans streptococci. Then, as the method for examining the caries risk without directly measuring the quantity of the mutans streptococci, an attention been attracted to the method, in which whether the antibody causing from a specific infection source is contained in a human body or not is examined for judging whether a person is infected with a specific infection or not. That is, since the antibody is produced in order to be against the specific infection source, it can be supposed that there is much quantity of the mutans streptococci for example, if the many antibodies against the mutans streptococci are contained in the human saliva. In fact, it has been known that the antibody against the mutans streptococci is secreted in the human saliva when a person is infected with the mutans streptococci, and it has been reported that an antibody value of an immunoglobulin against the S. mutans in the saliva is remarkably increased when the S. mutans is infected orally as a inactivated bacterial cell (for example, refer to a non-patent references, McGhee J. R. et al, Adv. Esp. Med. Biol. (USA), Plenum Press, 1978, 107, p.177-184 and Krasse B, L. et al, Adv. Exp. Med. Biol. (USA), Plenum Press, 1978, 107, p.349-354).

However, in a research that high or low of the antibody value of the saliva against the mutans streptococci contained in the saliva was used for judging the caries risk, a significant correlation between the given antibody value and the infecting situation of the caries or the quantity of the mutans streptococci in the saliva could not be found out, so that said research was not successful in applying it to the examination of the caries risk.

Recently, in a surface layer substance of the bacterial cell of the S. mutans being a kind of the mutans streptococci, it was confirmed that a protein antigen called as PAc (Protein Antigen cerotype C) with about 190000 molecular weight related with an initial adhesion of the mutans streptococci to the tooth surface in the research using the monoclonal antibody in which said protein antigen was used as the antigen. Based on the above confirmation, it was expected to find out the relationship between the PAc and the caries risk by measuring the antibody value in a blood plasma using the PAc being a part of said surface layer substance of the bacterial cell as the antigen instead of the mutans streptococci. However, in this case, the significant correlation between the PAc and the infecting situation of the caries or the quantity of the mutans streptococci in the saliva could not be found out, so that said research could not become the index for judging the risk.

As for the reason why there was no corrrelation between the antibody value using the mutans streptococci or the PAc and the quantity of the mutans streptococci, it was considered that a human immunoglobulin being produced for recognizing the other antigen was unexpectedly connected with a certain part of the mutans streptococci, since there were many antigen structure on the surface of the mutans streptococci or in the PAc. That is, it was considered that the human immunoglobulin, which is produced not for recognizing the mutans streptococci or the PAc, carried out a cross-reaction with the mutans streptococci or the PAc.

Then, in order to inhibit said cross-reaction, a research for specifying a certain part in the PAc, which purely related with the initial adhesion of the mutans streptococci to the tooth surface, had been advanced, and Senpuku being one of the present inventors et al., identified that an A area having a α spiral structure in the PAc (a part of the amino acid sequence 219-464) strongly influenced colonization and adhesion on the tooth surface by the S. mutans, (refer to a non-patent references,

Takahashi I. et al, "Immunogenicity and protective effect against oral colonization by Streptococcus mutans of synthetic peptides of a streptococcal surface protein antigen", J. Immunol, (USA), 1991, 146, p.332-6, Takahashi I. Infect Immun (USA), Baltimore Md. American Association of Immunologists, 1992, 60, p.623-629, and Okahashi N, et al, Mol. Microbiol. (USA) , Blackwell Scientific Publications, 1993, 3, p.221-228), and further, he solved what was the most important sequence in the A area of the PAc (refer to a non-patent reference Senpuku H, et al, "An antigenic peptide inducing cross-reacting antibodies inhibiting the interaction of Streptococcus mutans PAc with human salivary components" Infect Immun (USA) , Baltimore Md. American Association of Immunologists, 1995, 63, p.4695-4703). After that, it was identified that the sequence strongly acting on a human immune system as the antigen in the A area of the PAc was Y---L--Y, which was human B-cell epitope, and L--V-K--A, which was a part reacted with various human HLA-DR molecules, (refer to a non-patent reference, Senpuku et al, "Identification of Streptococcus mutans PAc peptide motif binding with human MHC class II molecules (DRBI * 0802, * 1101, * 1401, and * 1405) Immunology (England), Blackwell Scientific Publications, 1998, 95, p.322-330, and Senpuku et al, "Inhibitory Effects of MoAbs against a Surface Protein Antigen in Real-Time Adherence in vitro and Recolonization In vivo of Streptococcus mutans" Scand. J. Immunol. (England), Oxford Black well Scientific Publications, 2001, 54, p.109-116 ). From this results, the specific amino acid sequence in the PAc, i.e., [NAKATYEAALKQYEADLAAVKKANAA(PAC(361-386)}] was derived. As for the sequence of PAc (361-386), it was identified that a human anti-PAc (361-386) antibody could be induced by using this peptide from the experiment using a model mouse, (refer to a non-patent reference, Y. TSUHA, MD. A. SALAM, N. HANADA, N. KUROSAKI, and H. SENPUKU, 2800 Identification of peptide vaccine candidate to induce hu-antibody S.mutans PAc, IADR Poster, 2002/03/08), and the interaction with a caries experience could be identified from the antibody value in human plasma, (refer to a non-patent reference, Noboru Kaneko, Hidenobu Senpuku, NobuhiroHanada, and Hhideo Miyazaki, "Relation between an anti-PAc (361-386) antibody value in the blood plasma and DMFT in 80 years-old aged person", Journal of Dental Health, Vol 152, p.450-451, 2002). However, the correlation between this PAc and the quantity of the mutans streptococci in the oral cavity has not been given. Therefore, it is the present situation that the method for examining the caries risk, wherein a specific antigen is used and the caries risk can be accurately examined from the difference of the antibody value of the human immunoglobulin to the used specific antigen, is not established.

The object of the present invention is to provide a method for examining the caries risk, using a synthetic peptide having a specific amino acid sequence as the antigen, and accurately examining the caries risk from the difference of the antibody value of the human immunoglobulin to said antigen in short time.

The present inventors made wholeheartedly investigations in order to solve the above problems, and as the result, they considered that it was difficult from the characteristic of the antibody to obtain the quantity of the mutans streptococci in the oral cavity from the antibody value in the sequence of PAc (361-386) and the blood plasma, and that the necessary operation time of the examination using the blood plasma was from several hours to several days even if the correlation could be given, so that it could not be the method for examining the caries risk capable of being used clinically. Then, they noted that the motion of a mucosal immunity was different from that of the general immunity system, and invented that the examination of the caries risk could be accurately carried out in a short time, using the synthetic peptide having only the amino acid sequence of PAc (361-386) of the S. mutans as the antigen, and measuring the antibody value of the secretory immunoglobulin A (sIgA) secreted from the human oral cavity mucosa, which was working to inhibit the adhesion of the mutans streptococci to the tooth surface. Then, the present invention was completed.

The present invention is a method for examining the caries risk of a person, characterized by
using a synthetic peptide having the amino acid sequence composed of the following formula as the antigen, and measuring the antibody value of the secretory immunoglobulin A in the human saliva against said antigen.

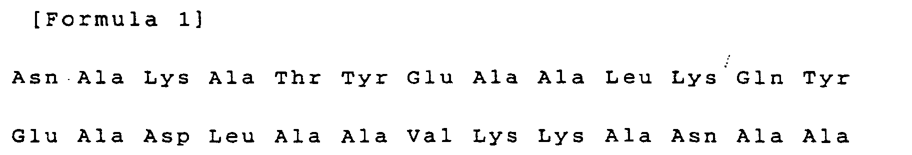

In order to give the synthetic peptides having the amino acid sequence composed of the above formula, which is used in the present invention as the antigen, although the method is not limited if said method can give said amino acid sequence, it is convenient to use an amino acid synthesizer in general. At the time of synthesizing the synthetic peptide , it is important that said synthetic peptide does not have an unnecessary amino acid sequence other than the amino acid sequence composed of the above formula. If there is an unnecessary amino acid sequence, the antibody value of the immunoglobulin being not originally related with the mutans streptococci is also measured, so that the examining accuracy is decreased.

In the present invention, the human saliva is used as a specimen, and the synthetic peptide having the amino acid sequence composed of the above formula is used as the antigen, and the antibody value of the secretory immunoglobulin A contained in the human saliva is measured. In fact, the specimen is arbitrary diluted with a physiological saline or a phosphate buffered saline. A person having the specimen, in which an antigen antibody reaction is given, even when having a high dilution ratio of the specimen, can be judged that he has a low caries crisis risk.

For quantitative determination of the antigen antibody reaction, the labeled antibody reacted with the secretory immunoglobulin A contained in the human saliva is used. As the labeling substance for said antibody, it is preferable that an enzyme, such as horseradish peroxidase and alkaline phosphatase, a fluorescent substance, such as fluorescein isothiocyanate, a gold colloid, or a latex bead, is used from view points of easiness in obtaining and labeling.

In the present invention, even if a conventional enzyme immunohistochemistry, i.e., an Enzyme Linked Immunosorbent Assay, which is referred to as "ELISA" hereinafter, being one of an antibody value measuring technique against the mutans streptococci, is used, sufficiently usable sensitivity can be given. However, the measurement sensitivity at the time of the determination can be increased by the technique conventionally used for improving the sensitivity of the measured value given by the antigen antibody reaction, for example, the method of reacting once the secretory immunoglobulin A in the saliva with a biotin anti-human immunoglobulin A etc. In order to measure the antibody value, the technique based on the general antigen antibody reaction can be applied as it is. In addition to said technique, each technique of an immuno-chromatography, an immuno-concentration, and a latex agglutination etc. can be used suitably.

In the present invention, it is important that the saliva is used as the specimen, and it is impossible to determine the quantity of the mutans streptococci if a human blood plasma is used.

As the method for measuring the antibody value of the secretory immunoglobulin A in the human saliva in the present invention, the measuring method, which has been conventionally used in immunology, can be used. For example, each method of the ELISA, the immuno-chromatography, the immuno-concentration, and the latex agglutination etc. can be used suitably.

At the time of measuring the antibody value, the synthetic peptide having the amino acid sequence composed of the above formula is formed into solid phase on a solid surface, and said solid phase is reacted with the arbitrary diluted specimen and the labeled antibody. Thus, the examination of the caries risk can be carried out.

At the time of forming into solid phase, the protein, which is not reacted with the specific antibody, can be coexisted for decreasing a background at the time of the measurement and stabilizing a solid-phase substance. As such protein, bovine serum albumin or skim milk, which are used in general, can be used. The skim milk ismore effective than the bovine serum albumin to give the lower background in many cases, so that it is more suitable.

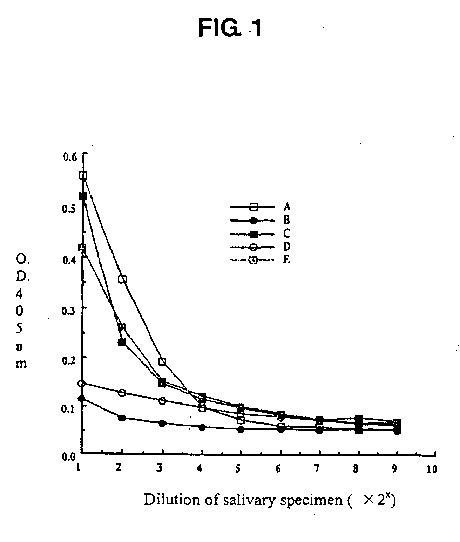

Figure 1 shows the results of measuring the antibody values against the synthetic peptide of the immunoglobulin A in saliva of 5 subjects (A, B, C, D, E) by the ELISA.

Hereinafter, the present invention will be explained concretely with reference to examples, but is not limited to the following examples. The operation was carried out at room temperature unless specifically described and pH is the value at 20 to 25 degree C. A concentration of protein quantity is calculated with absorbance of 280 nm wave length light.

### Example 1

### <Measurement of Salivation Antibody with ELISA>

### (1) Synthesis of the Synthetic peptide as Antigen

The protein having the amino acid sequence composed of the above formula was given by a stepwise solid-phase peptide synthetic method. Model 350 Multiple Peptide Synthesizer (a product name was Advanced Chemitech, produced by Louisville Company), was used as a synthesizer. The examination of the synthetic peptide was carried out by the high-performance reverse phase liquid chromatograph using TSK-GEL column (30 × 1), i.e., 10-45 % acetonitrile was used as column and gradient was 0.1 % TFA. The last refining degree was more than 95 %.

### (2) Measurement of Saliva Secretory Antibody

### A) Forming into solid phase of the antibody

The synthetic peptide synthesized by the above process was diluted with a 50mM sodium carbonate buffered solution having pH of 9.6 so as to be 10µ g / mL concentration, and this diluted protein was added to each well of a 96-wells microplate produced by Sumitomo Bakelite Company so as to become 100µ L. The added protein was kept quietly overnight at 4 degree C to bind with the well.

### B) Blocking

The microplate with the solid phase being formed by the above process was washed three times with a liquid (referred to as PBST hereinafter), in which 0.1 % by weight of neutral interfacial active agent (the product name was TWEEN20 produced by SIGMA Company) was contained in a PBS (Phosphate buffered saline having the pH of 7.4), by using a microplate washer (the product name was Model 1575 produced by Bio-Rat Company). After the washing, the PBS containing the 1 % skim milk was injected by 200 µ L per each well, and blocking was carried out at 37 degree C for one hour. After the blocking, washing was carried out three times with the PBST to wash an excessive blocking agent.

### C) Preparation of Saliva

A stimulated saliva secreted by chewing a paraffin wax for 3 minutes was used as the specimen. After obtaining, the salivary specimen was preserved in ice until starting the measurement. In the experiment, the saliva were prepared by diluting 2, 4, 8, 16, 32, 64, 128, 256, 512, and 1024 times with the PBST containing 0.5 % skim milk. Each of the saliva was added by 100 µ L to said plate. After adding, the plate was kept quietly at 37 degree C for one hour and then washed with the PBST.

### D) Labeled Antibody

An alkaline phosphatase label (the product name was Anti-Human-IgA, produced by Chemicon International Company), was prepared with the PBST containing the 0.5% by weight of skim milk so as to be the 0.3 µ g / mL concentration. 100 µ L of the prepared label was added to the each well to be reacted at 37 degree C for one hour.

### E) Coloring

After washing with the PBST, 100 µ m of a diethanolamine buffer containing 0.1% by weight of P-nitrophenol disodium phosphate hexahydrate was added to each well and kept quietly at 37 degree C for 30 minutes. Then, the absorbance 405 nm wavelength light was measured. In this case, the diethanolamine buffer was prepared with the following prescription.

| | |
|---|---|
| Diethanolamine | 48.5mL, |
| MgCl₂ · 6H₂O | 50.0mg |
| NaN₃ | 100mg, |
| H₂O | 400mL, |
| Final pH | 9.8. |

The antibody values of the immunoglobulin A in the saliva of five subjects (A, B, C, D, E) against the synthetic protein were measured with the ELISA, and these results were shown in Figure 1. As the result, it was identified that the examination condition of the present invention was the suitable condition for evaluating the antibody values. That is, by setting such condition grouping is made into two, i.e., the high antibody value group (A, C, E) and the low antibody value group (B,D), to make it possible that other subjects are examined with high or low evaluation according to the groups, or the caries risk can be examined with the actual value of the antibody value. In this time, the accuracy of the method for examining the caries risk was identified between the actual quantity of the mutans streptococci in the oral cavity and the groups of the antibody values being high and low.

### <Measurement of Mutans Streptococci>

### A)Preparation of Saliva

The stimulated saliva secreted by chewing the paraffin wax for 3 minutes was used as the specimen. After obtaining, the salivary specimen was immediately used for the following culture, and the remained specimen was preserved in ice.

### B) Measurement

The mutans streptococci in the saliva was measured by using MSB (Mitis Salivarius Bacitracin culture medium). As for the process, the saliva is diluted with the PBS under an aseptic condition and 50 µL are smeared on the culture medium. The smeared culture medium was cultured at 37 degree C under an anaerobic condition for 3 days. Then, the quantity of the mutans streptococci (cfu / mL) was calculated by measuring the number of colonies.

The antibody value measured by the method of the present invention was compared with the quantity of the mutans streptococci in the saliva measured with the colonies as shown in Table 1.

**Table 1**

| Antibody Value (O. D. 405 nm) | Subjects | Average of Quantity of Mutans Streptococci in Saliva (× 10⁵ cfu / mL) in Saliva (× 10⁵ cfu / mL) |
|---|---|---|
| High | A | 2.7 |
| | C | |
| | E | |
| Low | B | 15.5 |
| | D | |

According to the above results, it was identified that the antibody value of the secretory immunoglobulin A in the human saliva against the synthetic peptides has a correlation with the quantity of the mutans streptococci, and the examination of the caries risk can be carried out.

### Example 2

### <Method for Measuring Antibody value by Immuno-chromatography>

### A)Preparation of Porous Membrane smeared with trap-antibody >

A cellulose nitrate membrane (the product name was SXHF, produced by Nippon Millipore Company), was used as the porous membrane. Said membrane was cut out in a rectangle of 5mm × 40mm, and the 1 µL synthetic protein prepared to be 500 µ g / mL with the PBS was smeared on a central part of the membrane in a belt-shape. The smeared membrane was dried at 37 degree C for 2 hours and preserved in a desiccator until just before use.

### B) Preparation of Saliva

The stimulated saliva given by the subjects chewing the paraffin wax for 3 minutes was transferred into a plastic container, and the saliva was immediately diluted with the PBST 4 times and was used as the specimen.

### C) Measurement

A gold labeled anti-human IgA (the product name was BA. GAHA40, produced by British Biocell International Company) being diluted with the PBS 5 times was added by 50 µ L to the well of the 96-wells microplate, and the diluted salivary specimen was added by 100 µ L to said well to be mixed. The filter paper 40mm × 40mm being folded into four was fixed with clips at one end of the porous membrane smeared with the trap antibody prepared beforehand, and another end 5mmX40mm, where no filter paper is fixed, was dipped in the well to infiltrate the test liquid. Then, it was observed whether there is the antibody reaction or not.

### D) Measurement of Number of Mutans Streptococci

The quantity of the mutans streptococci was measured by the method described in Example 1.

The results were shown in Table 2. When a synthetic protein smeared part on the porous membrane, in which the trap antibody was smeared, was dyed in red, it was judged as "Reacted" in Table 2.

**Table 2**

| Reaction of Reaction of immuno-chromatography | Average Quantity of Mutans Streptococci in Saliva (× 10⁵ cfu / mL) |
|---|---|
| Reacted | 2.3 |
| Non-reacted | 16.5 |

The average of the quantity of the mutans streptococci of a group of "Non-reacted" (n = 42) was significantly higher (p = 0.05) than that of a group of "Reacted" (n = 25) . It was judged that the subject being "Non-reacted" in the present examination method has high caries risk.

### <Comparison example 1>

The antibody value of the saliva was examined by the same method of the ELISA shown in Example 1 excepting that the cultured S. mutans was formed into solid phase instead of the synthetic peptides. It was not identified that there is any correlation between the quantity of the mutans streptococci and the antibody value.

### <Comparison example 2>

A refined PAc was obtained, using a culture supernatant of a gene-recombinant S. mutans as a sample instead of the synthetic protein. The concrete refining method was according to the references, Okahashi N. et al, "Cloning of Surface protein antigen gene from serotype c Streptococcus mutans" Mol Microbiol, (USA), Blackwell Scientific Publications, 1989, 3. p.221 - 8, and
Koga T. et al, "Surface hydrophobicity, adherence, and aggregation of cell surface protein antigen mutans of Streptococcus mutans serotype c" , Infect Immum, (USA), Baltimore Md American Association of Immunologists, 1990, 58. p.289-96.
The examination was carried out by the same process as that of Example 2 excepting that the above refined PAc was used as the antigen. These results were shown in Table 3.

**Table 3**

| Reaction of immuno-chromatography | Average of Quantity of Mutans Streptococci in Saliva (× 10⁵ cfu / mL) |
|---|---|
| Reacted | 1.8 |
| Non-reacted | 1.7 |

There is no significant difference between the groups of "Reacted" and "Non-reacted" in the averages of quantity of the mutans streptococci (p = 0.05). Therefore, it could not be judged from Comparison example 2 that the caries risk was high or low.

As described above, the method for examining the caries risk of the present invention is the method capable of accurately examining the caries risk in a short time, by using as the antigen the synthetic peptides having the amino acid sequence peculiar to the mutans streptococci being the specific antigen, and measuring the difference of the antibody value of the secretory immunoglobulin A in the human saliva against said antigen. Therefore, this method has a great value for contributing to dental medicine.

## Claims

1. A method for examining a caries risk of a person,
**characterized by**
using a synthetic peptide having an amino acid sequence composed of a following Formula 1 as an antigen, and
measuring an antibody value of a secretory immunoglobulin A in a human saliva against said antigen.


SEQUENCE LISTING

<110> GC Corporation

<120> Method for Examining Caries Risk

<130> G 4267 EU

<140> EP 03020492.9
<141> 2003-09-15

<150> JP 2002-273125
<151> 2002-09-19

<160> 1

<170> PatentIn version 3.1

<210> 1
<211> 26
<212> PRT
<213> Artificial sequence

<220>
<223> Synthetic peptide

<400> 1

Asn Ala Lys Ala Thr Tyr Glu Ala Ala Leu Lys Gln Tyr Glu Ala Asp
1               5                   10                  15

Leu Ala Ala Val Lys Lys Ala Asn Ala Ala
            20                  25